# EUROPÄISCHE PATENTSCHRIFT

(11) **EP 0 576 905 B1**
(45) Veröffentlichungstag und Bekanntmachung des Hinweises auf die Patenterteilung: **03.01.1996**
(21) Anmeldenummer: 93109573.1
(22) Anmeldetag: 16.06.1993
(51) Int. Cl.: C07C 31/125

(54) **Gemische isomerer Decylalkohole, daraus erhältliche Phthalsäureester und ihre Verwendung als Weichmacher**
Mixtures of isomeric decyl alcohols, phthalic acid esters obtained therefrom and their use as plasticizers
Mélanges de décanols isomères, esters phthaliques obtenus à partir de ceux-ci leur utilisation comme plastifiants

(30) Priorität: 30.06.1992 DE 4221472
(43) Veröffentlichungstag der Anmeldung: 05.01.1994
(73) Patentinhaber: HOECHST AKTIENGESELLSCHAFT, D-65926 Frankfurt am Main (DE)
(72) Erfinder: Bahrmann, Helmut, Dr. Dipl.-Chem., D-4236 Hamminkeln 3 (DE); Greb, Wolfgang, Dr. Dipl.-Chem., D-4220 Dinslaken (DE); Heymanns, Peter, Dr. Dipl.-Chem., D-4300 Essen 1 (DE); Lappe, Peter, Dr. Dipl.-Chem., D-4220 Dinslaken (DE); Müller, Thomas, Dipl.-Ing., D-4220 Dinslaken (DE); Szameitat, Jürgen, Dr. Dipl.-Chem., 46487 Wesel (DE); Wiebus, Ernst, D-4200 Oberhausen 11 (DE)

(56) Entgegenhaltungen:
- EP-A- 0 052 999
- EP-A- 0 278 407
- EP-A- 0 366 089
- EP-A- 0 424 767
- WO-A-92/13818
- US-A- 4 982 031
- PATENT ABSTRACTS OF JAPAN, unexamined applications, C Sektion, Band 3, Nr. 109, 12. September 1979 THE PATENT OFFICE JAPANESE GOVERNMENT Seite 81 C 58

## Beschreibung

Die Erfindung betrifft Gemische isomerer Decylalkohole, ein Verfahren zu ihrer Herstellung, die aus diesen Alkoholen erhaltenen Phthalsäureester und deren Verwendung als Weichmacher.

Ester der Phthalsäure finden in großem Umfang als Weichmacher, insbesondere für Polyvinylchlorid, Verwendung. Als Alkoholkomponente werden vorwiegend primäre Alkohole mit 8 bis 10 Kohlenstoffatomen eingesetzt, größte Bedeutung unter ihnen hat gegenwärtig das 2-Ethylhexanol. Phthalsäureester kurzkettigerer Alkohole führen zu Weichmachern mit guter Gelierkraft. Nachteilig ist jedoch, verglichen mit längerkettigen Verbindungen, ihre höhere Flüchtigkeit. Längerkettige Ester gelieren dagegen langsamer und haben eine schlechtere Kältebeständigkeit.

Die Eigenschaften der Phthalsäureester-Weichmacher werden außer durch die Größe des Alkoholmoleküls auch durch die Verzweigung der Kohlenstoffkette beeinflußt. So ergeben wenig verzweigte Alkohole Esterweichmacher, die wegen ihrer hohen Kälteflexibilität besonders geschätzt sind. Weitgehend lineare Alkohole mit 9 bis 10 Kohlenstoffatomen im Molekül gewinnen daher als Alkoholkomponente zunehmende Bedeutung. Voraussetzung für ihren Einsatz ist, daß sie in großen Mengen und kostengünstig zur Verfügung stehen.

Nach der deutschen Patentschrift 2 855 421 werden als Weichmacher Phthalate von C₉-Alkoholen eingesetzt, die durch Oxoreaktion von C₈-Olefinen, Hydrierung des Reaktionsproduktes und Veresterung der C₉-Alkohole mit Phthalsäureanhydrid erhalten werden. 3 bis 20 Gew.-% der Ausgangsolefine sollen in jeder Molekülkette ein Isobutanskelett, weniger als 3 Gew.-% der Olefine quaternären Kohlenstoff aufweisen und mehr als 90 Gew.-% der Gesamtmenge der Olefine als n-Octene, Monomethylheptene und Dimethylhexene vorliegen. Ferner soll das Gewichtsverhältnis der Gesamtmenge der n-Octene und Monomethylheptene zu den Dimethylhexenen mehr als 0,8 betragen.

Phthalsäureester auf Basis von C₁₀-Alkoholen sind Gegenstand der europäischen Patentanmeldung 03 66 089. Die C₁₀-Alkohole werden in Form eines Gemisches eingesetzt, das man durch Hydroformylierung einer Butenfraktion, Aldolkondensation des erhaltenen Aldehydgemisches und anschließende Hydrierung gewinnt.

Ein anderer Weg zur Gewinnung von Di-Decylphthalatgemischen ist in der europäischen Patentanmeldung 04 24 767 beschrieben. Die Herstellung der Ester erfolgt nach einem mehrstufigen Verfahren durch Dimerisierung von Butengemischen, Hydroformylierung und Hydrierung des resultierenden Octengemisches zu einem Nonanolgemisch, Dehydratisierung des Nonanolgemisches unter Bildung eines Nonengemisches und Hydroformylierung und Hydrierung des Nonengemisches unter Bildung eines Decanolgemisches.

Die bekannten Verfahren erfüllen in wirtschaftlicher und technischer Hinsicht noch nicht alle Anforderungen, die an einen im großen Maßstab ausgeübten Prozeß gestellt werden, sei es, daß die Ausgangsstoffe nicht in ausreichender Menge und/oder nicht kostengünstig zur Verfügung stehen oder daß die Umwandlung der Ausgangsstoffe in die Alkohole an zu aufwendige Prozesse gebunden ist.

Es bestand daher die Aufgabe, ein Verfahren zu entwickeln, das von Rohstoffen ausgeht, die preiswert zur Verfügung stehen und die in technisch einfacher Weise in die gewünschten Alkohle überführt werden können.

Die Erfindung besteht in Gemischen isomerer Decylalkohole. Sie werden durch Oligomerisation von Propylen in Gegenwart desaktivierter Zeolithe als Katalysatoren, Abtrennung der C₉-Olefine aus dem Oligomerengemisch, Hydroformylierung der C₉-Olefine zu C₁₀-Aldehyden und Hydrierung der C₁₀-Aldehyde erhalten.

Die als Ausgangsmaterial zur Herstellung der erfindungsgemäßen Gemische isomerer Decylalkohole verwendeten C₉-Olefine sind in dem Produkt der Oligomerisation von Propylen in Gegenwart desaktivierter Zeolithe enthalten. Die Oligomerisation niedermolekularer Olefine wie Propylen, n-Buten, i-Buten, n-Penten unter der Einwirkung spezieller Zeolithe ist eine bekannte, z.B. in der US-Patentschrift 4 982 031 beschriebene Reaktion. Die Katalytisch wirksamen Zeolithe sind vorzugsweise vom ZSM-5-Typ, deren Oberfläche mit einer sterisch gehinderten Base, z.B. einem trialkylierten Pyridin desaktiviert wurde. Propylen als Olefin ergibt z.B. ein Oligomerengemisch, das sich im wesentlichen aus Monoolefinen mit 6, 9, 12, 15, 18 und 21 Kohlenstoffatomen zusammensetzt. Besonders bedeutsam ist, daß diese Olefine weitgehend unverzweigt sind. Bezogen auf die Verbindungen unterschiedlicher Molekülgröße im Oligomerengemisch entfallen auf 15 in unverzweigter Kette angeordnete Kohlenstoff-atome nur etwa ein bis zwei Methylverzweigungen. Durch den geringen Verzweigungsgrad unterscheiden sich die, wie vorstehend beschrieben gewonnenen C₉-Olefine erkennbar von dem als Tripropylen bezeichneten Propylenoligomeren, das aus Propylen in Gegenwart saurer Katalysatoren, insbesondere Phosphorsäure oder auch Schwefelsäure hergestellt wird und aus einem Gemisch stark verzweigter Nonene besteht.

Zur Gewinnung der für die Weiterverarbeitung vorgesehenen C₉-Olefine wird das Oligomerisationsprodukt auf konventionelle Weise destillativ in einzelne Fraktonen zerlegt. Die dabei anfallende Nonenfraktion kann ohne zusätzliche Reinigungsschritte hydroformyliert werden.

Die Hydroformylierung des erfindungsgemäß als Einsatzmaterial verwendeten C₉-Olefingemischs erfolgt nach den für Olefine gebräuchlichen Verfahren des Standes der Technik. Als Katalysatoren können in bekannter Weise sowohl Kobalt als auch Rhodium eingesetzt werden. Die Reaktion läßt sich in homogener flüssiger Phase, also mit Katalysatoren, die in organischen Medien löslich sind durchführen oder auch in heterogenen Reaktionssystemen, mit Katalysatoren, die in Wasser gelöst sind. Die zuletzt genannte Reaktionsweise hat sich insbesondere in Verbindung mit Rhodiumkatalysatoren bewährt.

Bei der Hydroformylierung in homogenen Reaktionssystemen arbeitet man nach dem klassischen Verfahren mit Kobaltkatalysatoren. Temperaturen von 90 bis 150°C, Drücke von 10 bis 30 MPa und Katalysatorkonzentrationen von 0,1 bis 1,5 Gew.-% Kobalt, bezogen auf eingesetztes Olefin sind die Standardreaktionsbedingungen. Statt Kobalt verwendet man als Hydroformylierungskatalysator auch Rhodium, das wesentlich aktiver als Kobalt ist und daher in geringerer Konzentration eingesetzt wird. Üblicherweise arbeitet man bei Temperaturen von 80 bis 200°C und Drücken von 10 bis 60 MPa mit 0,1 bis 50, vorzugsweise 2 bis 10 mg Rhodium je kg Olefin. Wirksame Katalysatorverbindung ist in beiden Fällen das Hydridocarbonyl HMe(CO)₄ mit Me = Co, Rh.

Statt die Umsetzung des Olefingemisches zu Aldehyden mit einfachen Carbonylverbindungen zu katalysieren verwendet man mit Erfolg auch modifizierte Carbonylverbindungen des Kobalts oder des Rhodiums. Als modifiziert bezeichnet man Carbonylverbindungen der genannten Metalle, die im Molekül neben Kohlenmonoxid noch weitere Komplexliganden enthalten. Auch diese Variante der Hydroformylierungsreaktions gehört sowohl bei Einsatz von Kobalt - als auch bei Einsatz von Rhodiumkatalysatoren zum Stand der Technik. Als Komplexliganden werden organische Verbindungen des dreiwertigen Phosphors bevorzugt. Beispiele für derartige Verbindungen sind Triarylphosphine wie Triphenylphosphin, Trialkylphosphine wie Tributylphosphin, Tri(alkyl/aryl)phosphine wie Diethylphenylphosphin, Triarylphosphite wie Triphenylphosphit, Trialkylphosphite wie Triethylphosphit, Tri(alkyl/aryl)phosphite und Phosphole. Bewährt haben sich auch zweizähnige Liganden, z.B. Diphosphine oder Diphosphite, die zwei zur Komplexbildung befähigte Phosphor(III)-Atome enthalten, unter diesen Verbindungen bevorzugt solche, die mit den Metallatomen Chelate bilden. Besonders bewährt haben sich zur Chelatbildung befähigte, sterisch gehinderte Phosphite als Liganden, die unter Einschluß des zentralen Metallatoms Ringe mit neun Atomen ergeben. Derartige Phosphite sind z.B. in der EP 0 353 770 A2 und in der EP 0 435 071 A2 beschrieben.

Üblicherweise werden die Liganden im Überschuß eingesetzt, also in einer Menge die die zur Komplexbildung erforderliche übersteigt. In der Praxis wählt man ein Verhältnis von Metallatom zu Ligandmolekül (in mol) von 1 : 5 bis 1 : 100. Es ist, wie bekannt u.a. von der Art des Liganden abhängig. Modifizierte Kobalt- bzw. Rhodiumkatalysaotren erlauben die Anwendung niedrigerer Reaktionsdrücke als unmodifizierte Katalysatoren, 0,1 bis 10 MPa, vzw. 0,5 bis 6 MPa sind gebräuchlich. Die Reaktionstemperaturen betragen bei Einsatz modifizierter Kobaltkatalysatoren 140 bis 180°C und liegen damit höher als bei den entsprechenden Rhodiumkatalysatoren, die Temperaturen von 90 bis 130°C erfordern. Das zur Hydroformylierung verwendete Synthesegas enthält etwa gleiche Volumina Wasserstoff und Kohlenmonoxid, jedoch sind Abweichungen von dieser Zusammensetzung in die eine oder die andere Richtung unschädlich.

Statt in homogener Phase kann die Hydroformylierung der C₉- Olefine auch in einem heterogenen Zweiphasensystem erfolgen, eine Ausführungsform der Oxosynthese, die z.B. in der DE-PS 26 27 354 beschrieben ist. Ein solcher Prozeß ist charakterisiert durch das Vorliegen zweier flüssiger Phasen, einer organischen Phase, die das Ausgangsolefin und das Reaktionsprodukt enthält und einer wäßrigen Phase, in der der Katalysator gelöst ist. Als Katalysatoren finden wasserlösliche Rhodiumkomplexverbindungen mit wasserlöslichen Phosphinen als Liganden Anwendung. Zu den wasserlöslichen Phosphinen zählen insbesondere Triarylphosphine, Trialkylphosphine und Alkyl-, Aryl- oder Alkylaryldiphosphine, deren organische Reste durch Sulfonsäuregruppen oder Carboxylgruppen substituiert sind. Die Reaktion erfolgt bei Temperaturen von 60 bis 150°C, vorzugsweise 90 bis 120°C und unter Drücken im Bereich von 0,4 bis 30, insbesondere 1 bis 10 MPa. Die Rhodiumkonzentration beträgt 20 bis 2000 Gew.-ppm, vorzugsweise 50 bis 500 Gew.-ppm, bezogen auf die wäßrige Katalysatorlösung, je mol Rhodium setzt man 4 bis 100 mol wasserlösliches Phosphin ein. Das Volumverhältnis von wäßriger zu organischer Phase beträgt 0,1 bis 10 : 1.

Der Olefinumsatz wird deutlich erhöht, wenn man der wäßrigen Katalysatorlösung ein Phasentransferreagenz (Lösungsvermittler) zusetzt. Bewährt haben sich insbesondere kationische Lösungsvermittler der allgemeinen Formel [A-N(R¹R²R³)]⁺E⁻, in der A für einen geradkettigen oder verzweigten Alkylrest mit 6 bis 25 Kohlenstoffatomen steht, R¹, R², R³ gleich oder verschieden sind und geradkettige oder verzweigte Alkylreste mit 1 bis 4 Kohlenstoffatomen bedeuten und E insbesondere für Sulfat, Tetrafluorborat, Acetat, Methosulfat, Benzolsulfonat, Alkylbenzolsulfonat, Toluolsulfonat, Lactat oder Citrat steht.

Die wahlweise im einphasigen oder im zweiphasigen Reaktionssystem durchgeführte Hydroformylierung der erfindungsgemäß eingesetzten C₉-Olefine mit Kobalt oder Rhodium als Katalysator liefert in hoher Ausbeute ein Gemisch isomerer Decanale. Es enthält 80 und mehr Prozent geradkettigen C₁₀-Aldehyd und nur in untergeordnetem Maße methyl- und dimethylverzweigte Aldehyde. Die Zusammensetzung des Aldehydgemischs kann durch Variation der Reaktionsbedingungen und/oder des Katalysators, insbesondere durch Wahl des Liganden bei der Verwendung modifizierter Carbonyle beeinflußt werden.

Nach Beendigung der Hydroformylierung wird das Aldehydgemisch vom Katalysator, von den nichtumgesetzten Reaktionsteilnehmern und den übrigen Reaktionsprodukten abgetrennt. Bei Umsetzung in homogener Phase ist die Destillation das übliche Trennverfahren. Wurde die Hydroformylierung in einem Zweiphasensystem durchgeführt, dann können Produkt und Katalysator durch einfache Phasentrennung voneinander geschieden werden. Dieses Verfahren ist in der technischen Durchführung wesentlich einfacher und wegen der fehlenden thermischen Belastung auch erheblich schonender als die destillative Isolierung des Aldehydgemischs.
Die Hydroformylierung des C₉-Olefingemischs kann, unabhängig vom angewandten Prozeß, diskontinuierlich oder kontinuierlich erfolgen.

Die von den übrigen Bestandteilen des Reaktionsgemisches abgetrennten isomeren Decanale werden anschließend zu einem Decylalkoholgemisch hydriert. Die Wasserstoffanlagerung erfolgt in bekannter Weise in Gegenwart von Katalysatoren. Geeignet sind z.B. Hydrierkatalysatoren auf Basis von Nickel, Chrom oder Kupfer. Üblicherweise liegt die Hydriertemperatur zwischen 100 und 180°C und der Druck zwischen 1 und 10 MPa. Zur Reinigung wird das Decylalkoholgemisch destilliert. Es eignet sich vorzüglich als Alkoholkomponente in Phthalsäureestern, die als Weichmacher verwendet werden sollen. Die Herstellung der Phthalsäureester ist bekannt [vgl. Ullmann, Encyclopädie der Technischen Chemie (1979), Bd. 18, Seite 536 ff]. Zweckmäßig setzt man Phthalsäureanhydrid mit dem Decylalkoholgemisch im Molverhältnis 1 : 2 in einer Stufe um. Die Reaktionsgeschwindigkeit kann durch Katalysatoren und/oder durch Erhöhung der Reaktionstemperatur erhöht werden. Um das Gleichgewicht in Richtung der Esterbildung zu verschieben, ist es erforderlich, das gebildete Wasser aus dem Reaktionsgemisch zu entfernen.

Die aus dem erfindungsgemäßen Decylalkoholgemisch erhaltenen Phthalate zeichnen sich als Weichmacher durch hervorragende Kälteeigenschaften aus.

In dem nachfolgenden Beispiel wird die Erfindung näher erläutert. Sie ist jedoch nicht auf diese spezielle Ausführungsform beschränkt.

### Beispiel

### 1. Ausgangsolefin

Es wird ein durch Umsetzung von Propylen an einem desaktivierten Zeolith als Katalysator erhaltenes Oligomerpropylen eingesetzt, das zu etwa 87,3 Gew.-% aus Tripropylen und 12,7 Gew.-% Tetrapropylen besteht. Die Olefine sind zu einem hohen Anteil linear, eine geringe Menge ist wenig verzweigt.

### 2. Hydroformylierung

500 g des Olefingemisches werden in einem 1 l-Autoklaven in Gegenwart von 10 g CoCO₃ und 15 g Wasser bei 170°C und 27 MPa 4 Stunden mit Wassergas (H₂:CO = 1:1) umgesetzt. Zu dem entspannten und abgekühlten Autoklaveninhalt gibt man 52 g Wasser. Der Autoklav wird erneut verschlossen und unter Rühren 2 Stunden auf 190°C erhitzt. Man kühlt ab und trennt organische und wäßrige Phase voneinander. Es werden 549 g Rohaldehyd erhalten.

### 3. Hydrierung des Rohaldehyds

Der Rohaldehyd wird in einem 2,8 l-Autoklaven bei 150°C und unter einem Druck von 10 MPa in Gegenwart eines Nickelkatalysators (10 Vol.-%, bezogen auf das Einsatzprodukt) mit Wasserstoff behandelt. Darauf kühlt man ab, entspannt und hydriert nochmals unter Zusatz von weiteren 5 Vol.-% Nickelkatalysator unter den gleichen Reaktionsbedingungen. Der Reaktor wird abgekühlt und entspannt und das Produkt durch Filtration vom Nickelkatalysator getrennt.

### 4. Destillation des Rohalkohols

825,5 g Rohalkohol werden anschließend in einer mit 5 mm Glasringen versehenen 1 m Kolonne destilliert. Bei 100°C Kopftemperatur und 13,3 kPa Druck wird ein Vorlauf, bei 135°C und 6,7 kPa Druck ein Zwischenlauf abgenommen. Die Hauptfraktion erhält man bei 147°C Kopftemperatur und 6,7 kPa. Die gaschromatographische Untersuchung ergibt einen Alkoholgehalt von 95,7 Gew.-%. Die Ausbeute an C₁₀-Alkohol, bezogen auf das Einsatzolefin, beträgt 72 % der Theorie.

### 5. Veresterung des Reinalkohols

380,4 g (2,3 mol) C₁₀-Alkohol und 148,1 g (1 mol) Phthalsäureanhydrid werden in Gegenwart von 0,59 g konzentrierter Schwefelsäure bei 135°C unter Abtrennung des anfallenden Reaktionswassers umgesetzt. Die Reaktion ist nach 6 Stunden beendet, das Reaktionsprodukt wird mit 5 %iger Natronlauge neutralisiert. Anschließend führt man bei 135°C und 15 kPa eine Wasserdampfdestillation durch. Das Destillat wird mit Wasser gewaschen und darauf 5 Stunden im Stickstoffstrom bei 135°C und 2 bis 3 kPa getrocknet. Nach Filtration erhält man eine klare Flüssigkeit, die durch die nachstehenden Kenndaten charakterisiert ist

| | |
|---|---|
| Estergehalt | 99,41 Gew.-% |
| Alkoholgehalt | 0,22 Gew.-% |
| Pt-Co-Farbzahl | 5 - 10 |
| Dichte (g/ml; 20°C) | 0,9655 |
| Viskosität (mPa.s; 20°C) | 108 |
| Säurezahl (mgKOH/g) | 0,06 |

Die Viskosität des Esters liegt deutlich niedriger als die Viskosität gängiger Produkte auf Basis üblicher Polymerpropylene, die 120 bis 130 mPa.s/20°C beträgt. Er zeichnet sich daher durch hervorragende verarbeitungstechnische Eigenschaften aus.

## Patentansprüche

1. Gemische isomerer Decylalkohole erhalten durch Oligomerisation von Propylen in Gegenwart desaktivierter Zeolithe als Katalysatoren, Abtrennung der C₉-Olefine aus dem Oligomerengemisch, Hydroformylierung der C₉-Olefine zu C₁₀-Aldehyden und Hydrierung der C₁₀-Aldehyde.

2. Gemische isomerer Decylalkohole nach Anspruch 1, dadurch gekennzeichnet, daß die Hydroformylierung der C₉-Olefine in Gegenwart von Kobaltkatalysatoren erfolgt.

3. Gemische isomerer Decylalkohole nach Anspruch 1, dadurch gekennzeichnet, daß die Hydroformylierung der C₉-Olefine in Gegenwart von Rhodiumkatalysatoren erfolgt.

4. Gemische isomerer Decylalkohole nach Anspruch 1 oder 3, dadurch gekennzeichnet, daß die Hydroformylierung der C₉-Olefine in Gegenwart von Rhodiumkomplexverbindungen als Katalysatoren erfolgt, die organische Phosphine als Liganden enthalten.

5. Gemische isomerer Decylalkohole nach einem oder mehreren der Ansprüche 1,3 oder 4, dadurch gekennzeichnet, daß die Hydroformylierung in Gegenwart von Rhodiumkomplexverbindungen als Katalysatoren erfolgt, die wasserlösliche organische Phosphine als Liganden enthalten.

6. Gemische isomerer Decylalkohole nach Anspruch 1 oder 3 dadurch gekennzeichnet, daß die Hydroformylierung der C₉-Olefine in Gegenwart von Rhodiumkomplexverbindungen als Katalysatoren erfolgt, die organische Phosphite als Liganden enthalten.

7. Gemische isomerer Decylalkohole nach einem oder mehreren der Ansprüche 1,3 oder 6, dadurch gekennzeichnet, daß die Hydroformylierung der C₉-Olefine in Gegenwart von Rhodiumkomplexverbingdungen erfolgt, die sterisch gehinderte organische Phosphite als Liganden enthalten.

8. Gemische isomerer Decylphthalate erhalten durch Veresterung von Phthalsäure oder Phthalsäureanhydrid mit Gemischen isomerer Decylalkohole nach einem oder mehreren der Ansprüche 1 bis 7.

9. Verwendung der Gemische isomerer Di-decylphthalate nach Anspruch 9 als Weichmacher.

## Claims

1. A mixture of isomeric decyl alcohols obtained by oligomerization of propylene in the presence of deactivated zeolites as catalysts, separation of the C₉-olefins from the oligomer mixture, hydroformylation of the C₉-olefins to C₁₀-aldehydes, and hydrogenation of the C₁₀-aldehydes.

2. A mixture of isomeric decyl alcohols as claimed in claim 1, wherein the hydroformylation of the C₉-olefins is carried out in the presence of cobalt catalysts.

3. A mixture of isomeric decyl alcohols as claimed in claim 1, wherein the hydroformylation of the C₉-olefins is carried out in the presence of rhodium catalysts.

4. A mixture of isomeric decyl alcohols as claimed in claim 1 or 3, wherein the hydroformylation of the C₉-olefins is carried out in the presence of rhodium complex compounds as catalysts, which contain organic phosphines as ligands.

5. A mixture of isomeric decyl alcohols as claimed in one or more of claims 1, 3 and 4, wherein the hydroformylation is carried out in the presence of rhodium complex compounds as catalysts, which contain water-soluble organic phosphines as ligands.

6. A mixture of isomeric decyl alcohols as claimed in claim 1 or 3, wherein the hydroformylation of the C₉-olefins is carried out in the presence of rhodium complex compounds as catalysts, which contain organic phosphites as ligands.

7. A mixture of isomeric decyl alcohols as claimed in one or more of claims 1, 3 and 6, wherein the hydroformylation of the C₉-olefins is carried out in the presence of rhodium complex compounds that contain sterically hindered organic phosphites as ligands.

8. A mixture of isomeric decyl phthalates obtained by esterifying phthalic acid or phthalic anhydride with mixtures of isomeric decyl alcohols as claimed in one or more of claims 1 to 7.

9. The use of the mixtures of isomeric di-decyl phthalates as claimed in claim 9 as plasticizers.

## Revendications

1. Mélanges de décanols isomères obtenus par oligomérisation du propylène en présence de zéolites désactivées qui servent de catalyseurs, séparation des oléfines en C₉ à partir du mélange des oligomères, hydroformylation des oléfines en C₉ en aldéhydes en C₁₀ et hydrogénation des aldéhydes en C₁₀.

2. Mélanges de décanols isomères selon revendication 1, caractérisés en ce que l'hydroformylation des oléfines en C₉ est réalisée en présence de catalyseurs au cobalt.

3. Mélanges de décanols isomères selon la revendication 1, caractérisés en ce que l'hydroformylation des oléfines en C₉ est réalisée en présence de catalyseurs au rhodium.

4. Mélanges de décanols isomères selon la revendication 1 ou 3, caractérisés en ce que l'hydroformylation des oléfines en C₉ est réalisée en présence de catalyseurs consistant en complexes du rhodium qui contiennent des phosphines organiques en tant que ligands.

5. Mélanges de décanols isomères selon une ou plusieurs des revendications 1, 3 ou 4, caractérisés en ce que l'hydroformylation est réalisée en présence de catalyseurs consistant en complexes du rhodium qui contiennent en tant que ligands des phosphines organiques solubles dans l'eau.

6. Mélanges de décanols isomères selon la revendication 1 ou 3, caractérisés en ce que l'hydroformylation des oléfines en C₉ est réalisée en présence de catalyseurs consistant en complexes du rhodium dont les ligands sont des phosphites organiques.

7. Mélanges de décanols isomères selon une ou plusieurs des revendications 1, 3 ou 6, caractérisés en ce que l'hydroformylation des oléfines en C₉ est réalisée en présence de complexes du rhodium contenant, en tant que ligands, des phosphites organiques faisant l'objet d'un empêchement stérique.

8. Mélanges de phtalates de décyle isomères obtenus par estérification de l'acide phtalique ou de l'anhydride phtalique par des mélanges de décanols isomères selon une ou plusieurs des revendications 1 à 7.

9. Utilisation des mélanges de phtalates de didécyle isomères selon la revendication 9 en tant que plastifiants.
